# EUROPEAN PATENT SPECIFICATION

(11) **EP 0 415 731 B1**
(45) Date of publication and mention of the grant of the patent: **02.04.2003**
(21) Application number: 90309430.8
(22) Date of filing: 29.08.1990
(51) Int. Cl.: C12N 15/85, C12N 15/52, C12N 15/86, C12N 7/01, A61K 48/00, A61K 31/70

(54) **Novel entities for cancer therapy**
Neue Substanzen für die Krebstherapie
Substances nouvelles pour la thérapie du cancer

(30) Priority: 30.08.1989 GB 8919607
(43) Date of publication of application: 06.03.1991
(60) Divisional application: 95100248.4
(73) Proprietor: THE WELLCOME FOUNDATION LIMITED, Greenford, Middlesex UB6 0NN (GB)
(72) Inventor: Huber, Brian, Durham, NC 27713 (US); Richards, Cynthia Ann, Durham, NC 27713 (US); Krenitsky, Thomas Anthony, Chapel Hill, NC 27514 (US)
(74) Representative: Rees, Marion Lindsay

(56) References cited:
- EP-A- 0 272 065
- EP-A- 0 293 193
- EP-A- 0 294 114
- WO-A-89/07136
- PROCEEDINGS OF THE NATL. ACADEMY OF SCIENCES USA, vol. 86, April 1989, Washington (US); R.D. HEYMAN et al., pp. 2698-2702

## Description

The present invention relates to molecular chimaeras, infective virions, to methods of their construction, to pharmaceutical formulations containing them, to their use in therapy, particularly virus-directed enzyme prodrug therapy, particularly in the treatment of cancers, more particularly in the treatment of hepatocellular carcinoma, and to the use of agents which can be catalysed by a heterologous enzyme to cytotoxic or cytostatic metabolites, such as purine arabinosides and substituted pyrimidines in virus-directed enzyme prodrug therapy.

Cancer of all forms is one of the major causes of morbidity throughout the world. Research in the area of cancer chemotherapy has produced a variety of antitumour agents which have differing degrees of efficacy. Standard clinically used agents include adriamycin, actinomycin D, methotrexate, 5-fluorouracil, cis platinium, vincristine and vinblastine. However, these presently available antitumour agents are known to have various disadvantages such as toxicity to healthy cells and resistance of certain tumour types. Other forms of therapy such as surgery, are known. However it is appreciated by those skilled in the art that novel approaches and entities for cancer therapies are required.

Hepatocellular carcinoma (HCC) is one of the major malignant diseases in the world today; the greatest incidence being in Japan, China, other parts of Asia and sub-Saharan Africa. Recent evidence suggests that the incidence of hepatocellular carcinoma in Europe and North America is increasing. The disease is estimated to be responsible for or involved in up to approximately 1,250,000 deaths a year and as such is numerically, one of the world's major malignant diseases.

The prognosis of HCC is always poor with the world-wide frequency rate almost equalling the mortality rate. After diagnosis, the median survival time is less than four months. Long-term survival, defined as survival longer than one year after diagnosis, is seen only occasionally. Most HCC patients succumb to either the complications of liver failure with or without massive bleeding, or to the general effects of a large tumour burden, with cachexia, malnutrition, infection and sepsis. Though distant metastases occur (up to 90% of patients have metastatic tumour at time of death), regional disease most often limits survival. Consequently, therapies directed towards control of hepatic tumour are appropriate although it will be appreciated that treatment of the metastatic disease is also of great importance. (Kew M.C. Postgraduate Medical Journal 59 (Suppl.) 78-87 (1983) and Berk.P. (Ed) Seminars in Liver Disease 4, No.2, Thieme-Stratton Inc. N.Y. N.Y. (1984)).

Current therapies available to the clinician are on the whole ineffective as a cure for this disease (Nerenstone *et al,* Cancer Treatment Reviews 15, 1-31 (1988)). To date, surgery continues to be the only potential cure. However, at the time of diagnosis, the overwhelming majority of patients are not able to undergo radical surgery. In certain studies (Nerenstone *et al* supra) it was found that less than 3% of patients were considered capable of undergoing surgery and of the small percentage that so do approximately 50% suffer from postoperative morbidity (Nerenstone *et al* supra).

Systemic single and combination agent chemotherapy and radiation are relatively ineffective and this emphasises the need for new approaches and therapies for the treatment of this disease.

Gene therapy involves the stable integration of new genes into particular target cells and the expression of those genes, once they are in place, to alter the phenotype of that particular target cell (for review see Anderson, W.F. Science 226: 401-409, 1984; McCormick, D. Biotechnology 3 : 690-693, 1985). Gene therapy may be beneficial for the treatment of genetic disease which involve the replacement of one defective or missing enzyme, such as hypoxanthine-guanine phosphoribosyl transferase in Lesch Nyhan disease; purine nucleoside phosphorylase in severe immunodeficiency disease and adenosine deaminase in severed combined immunodeficiency disease. As yet, gene therapy has not been used in the clinic, although in certain disorders, certain types of gene therapy are likely to be used imminently.

It has been found by the present inventors that it is possible to selectively arrest the growth of or kill mammalian carcinoma cells with chemical agents capable of selective conversion to cytotoxic or cytostatic metabolites. This is achieved by the construction of a molecular chimaera comprising a "target tissue-specific" transcriptional regulatory sequence (TRS) which is selectively activated in cancerous cells: this controls the expression of a heterologous enzyme. This molecular chimaera may be manipulated via suitable vectors and incorporated into an infective virion. Upon administration of an infective virion containing the molecular chimaera to a patient, the enzyme is selectively expressed in the target cell. Administration of compounds which are selectively metabolised by the enzyme into metabolites which are either further metabolised to, or are infact cytotoxic or cytostatic agents can then be achieved *in situ*.

The invention is generally applicable and is demonstrated with respect to hepatocellular carcinoma.

As mentioned above the overwhelming percentage of patients suffering from hepatocellular carcinoma die from the primary tumour. However, approximately 90% of HCC patients have overt metastatic disease at time of death. These metastases exhibit the typical phenotype of the primary tumour and as such these metastases will also selectively express the heterologous enzyme and thus selectively activate administered compounds as herein defined to cytotoxic or cytostatic metabolites.

A number of enzyme pro-drug combinations may be used: providing the enzyme is capable of selectively activating the administered compound either directly or through an intermediate to a cytostatic or cytotoxic metabolite. Equally the choice of compound will depend on the enzyme system used, but must be selectively metabolised by the enzyme either directly or indirectly to a cytotoxic or cytostatic metabolite. The term heterologous enzyme as used herein, refers to an enzyme which displays the appropriate characteristics of selectivity.

The varicella zoster virus (VZV) encodes a specific thymidine kinase protein. The gene has been cloned, sequenced and characterised (J. Gen. Virol. 67 : 1759-1816 (1986)). The VZV thymidine kinase will, in contrast to the mammalian enzyme, selectively monophosphorylate specific purine arabinosides and substituted pyrimidine compounds. Moreover, the present inventors have found that certain purine and pyrimidine analogues particularly those of formulae (I) and (II) as hereinafter defined are converted to cytotoxic or cytostatic metabolites in specific mammalian cells which are genetically modified to selectively express VZV thymidine kinase. For example 9-(β-D-arabinofuranosyl)-6-methoxy-9H-purine is converted to 9-β-D-arabinofuranosyl adenine triphosphate [Ara ATP].

Other enzyme pro-drug combinations include the bacterial (for example from Pseudomonas) enzyme carboxypeptidase G2 with the prodrug para-N-bis (2-chloroethyl) aminobenzoyl glutamic acid. Cleavage of the glutamic acid moiety from this compound releases a toxic benzoic acid mustard: alkaline phosphotase from, for example, calf intestine, will convert inactive phosphorylated compounds such as etoposide-phosphate, doxorubicin-phosphate, mitomycin phosphate, to toxic dephosphorylated metabolites. Penicillin-V amidase will convert phenoxyacetamide derivatives of doxorubicin and melphalan to toxic metabolites and the fungal (for example from Fusarium oxysporum) cytosine deaminase will convert 5-fluorocytosine to toxic 5-fluorouracil.

In mammalian cells, certain genes are ubiquitously expressed. Most genes, however, are expressed in a temporal and/or tissue-specific manner, or are activated in response to extracellular inducers, for example certain genes are actively transcribed only at very precise times in ontogeny in specific cell types or in response to some inducing stimulus. This regulation is mediated in part by the interaction between transcriptional regulatory sequences (which are for example promoter and enhancer regulatory DNA sequences), and sequence-specific, DNA-binding transcriptional protein factors.

The present inventors have found that it is possible to alter certain mammalian tissues, eg. liver tissue or transformed liver tissue, to selectively express a heterologous enzyme as herein before defined, eg. VZV thymidine kinase. This is achieved by the construction of molecular chimaeras in an expression cassette.

Expression cassettes themselves are well known in the art of molecular biology. Such an expression cassette will contain all essential DNA sequences required for expression of the heterologous enzyme in a mammalian cell. For example, a preferred expression cassette will contain a molecular chimaera containing the coding sequence for VN TK, an appropriate polyadenylation signal for a mammalian gene (i.e. a polyadenylation signal which will function in a mammalian cell), and suitable enhancers and promoter sequences in the correct orientation.

In mammalian cells, normally two DNA sequences are required for the complete and efficient transcriptional regulation of genes that encode messenger RNAs in mammalian cells: promoters and enhancers. Promoters are located immediately upstream (5') from the start site of transcription. Promoter sequences are required for accurate and efficient initiation of transcription. Different gene-specific promoters reveal a common pattern of organisation. A typical promoter includes an AT-rich region called a TATA box (which is located approximately 30 base pairs 5' to the transcription initiation start site) and one or more upstream promoter elements (UPE). The UPEs are a principle target for the interaction with sequence-specific nuclear transcriptional factors. The activity of promoter sequences is modulated by other sequences called enhancers. The enhancer sequence may be a great distance from the promoter in either an upstream (5') or downstream (3') position. Hence, enhancers operate in an orientation- and position-independent manner. However, based on similar structural organisation and function that may be interchanged the absolute distinction between promoters and enhancers is somewhat arbitrary. Enhancers increase the rate of transcription from the promoter sequence. It is predominantly the interaction between sequence specific transcriptional factors with the UPE and enhancer sequences that enable mammalian cells to achieve tissue-specific gene expression. The presence of these transcriptional protein factors (tissue-specific, trans-activating factors) bound to the UPE and enhancers (cis-acting, regulatory sequences) enable other components of the transcriptional machinery, including RNA polymerase, to initiate transcription with tissue-specific selectivity and accuracy.

The selection of the transcriptional regulatory sequence, in particular the promoter and enhancer sequence will depend on the targeted tissue. Examples include, the alpha-fetoprotein (AFP) transcriptional regulatory sequence (for example, the promoter and enhancer) specific for transformed hepatocytes; the transcriptional regulatory sequence for carcinoembryonic antigen (CEA) for use in transformed cells of the gastrointestinal tract, lung, breast and other tissues.

In addition the transcriptional regulatory sequences for certain oncogenes may be used as these are expressed predominantly in certain tumour types. Good examples of these include the HER-2/neu oncogene regulatory sequence which is expressed in breast tumours.

The ALB and AFP gene exhibits extensive homology with regard to nucleic acid sequence, gene structure, amino acid sequence and protein secondary folding (for review see Ingram *et al* PNAS 78 4694-4698 (1981). These genes are independently but reciprocally expressed in ontogeny. In normal development ALB transcription is initiated shortly before birth and continues throughout adulthood. Transcriptional expression of ALB in the adult is confined to the liver. AFP is normally expressed in fetal liver, the visceral endoderm of the yolk sac and the fetal gastrointestinal tract, but declines to undetectable levels shortly after birth and is not significantly expressed in nonpathogenic or non-regenerating adult liver or in other normal adult tissues. However, AFP transcription in adult liver often increases dramatically in HCC. In addition, transcription may also be elevated in non-seminomatous and fixed carcinoma of the testis; in endodermal sinus tumours, in certain tertocarcinomas, and in certain gastrointestinal tumours. Liver-specific expression of AFP is the result of interactions of the regulatory sequences of their genes with trans-activating transcriptional factors found in nuclear extracts from liver. The AFP transcriptional regulatory sequence is preferred for generating hepatoma-specific expression of molecularly combined genes since the AFP gene is regulated at the transcriptional level and the mRNA is among the most abundant polymerase II transcripts in the liver.

Several mammalian AFP promoter and enhancer sequences have been identified (for review see Genes and Develop. 1: 268-276 (1987); Science 235 : 53-58 (1987); The Journal of Biol.Chemistry 262 : 4812-4818 (1987). These sequences enable the selective and specific expression of genes in hepatomas.

The regulatory elements of the AFP genes promote tissue-specific expression of AFP in certain liver pathologies, such as HCC (Hol.Cel.Biol. 6 : 477-487 (1986); Science 235: 53-58 (1987)). The regulatory elements of a mammalian AFP gene consist of a specific 5' promoter-proximal region (located in some mammalian species between 85 and 52 bp 5' to the gene). This sequence is essential for transcription in hepatomas. In addition, there are upstream (5') regulatory elements well defined for the murine AFP gene which behave as classical enhancers (Mol.Cel.Biol. 6 : 477-487 (1986); Science 235: 53-58 (1987)). These upstream regulatory elements are designated elements I, II, and III and are located between 1,000 to 7,600 bp 5' to the transcription initiation site for the AFP murine gene. These three enhancer domains are not functionally equivalent at generating tissue-specific expression of AFP. Elements I and II have the greatest capacity to direct liver-specific expression of AFP. It is important to note that the regulatory sequences of the alpha-fetoprotein gene advantageously contain the sequences not only for tissue-specific transcriptional activation but also for repression of expression in tissues which should not express AFP. In a similar fashion the regulatory regions of the human alphafetoprotein gene have been characterised (J.B.C. 262 4812 (1987)). A structural gene placed in the correct orientation 3' to the AFP regulatory sequences will enable that structural gene to be selectively expressed in fetal liver, hepatomas, non seminamatous carcinomas of the testis, certain teratocarcinomas, certain gastrointestinal tumours and other normal and pathological tissues which specifically express AFP.

The present invention provides a molecular chimaera comprising a DNA sequence containing the coding sequence of the gene coding for a heterologous enzyme under the control of a transcriptional regulatory sequence in an expression cassette; the promoter capable of functioning selectively in a target cancer cell; for example one which is capable of transforming a cancer cell to selectively express thymidine kinase.

The present invention further provides in a preferred embodiment, a molecular chimaera comprising a transcriptional regulatory sequence, in particular a promoter which is selectively activated in mammalian target tissues and operatively linked to the coding sequence for the gene encoding varicella zoster virus thymidine kinase (VZV TK).

In particular, the present invention provides a molecular chimaera comprising a DNA sequence of the coding sequence of the gene coding for the enzyme, which is preferably VZV TK, including an appropriate polyadenylation sequence, linked in a 3' position and in the proper orientation to a mammalian target tissue specific transcriptional regulatory sequence. Most preferably the expression cassette also contains an enhancer sequence.

The promoter and enhancer sequences, preferably, are selected from the transcriptional regulatory sequence for one of alphafetoprotein (AFP), carcinoembryonic antigen (CEA), or HER2/neu or other suitable genes. Most preferably the regulatory sequence for AFP is used to direct hepatoma specific expression.

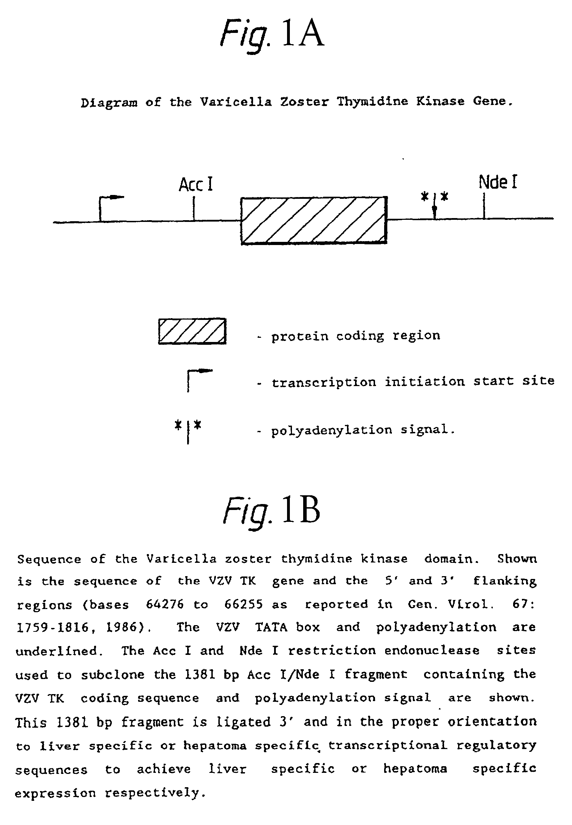

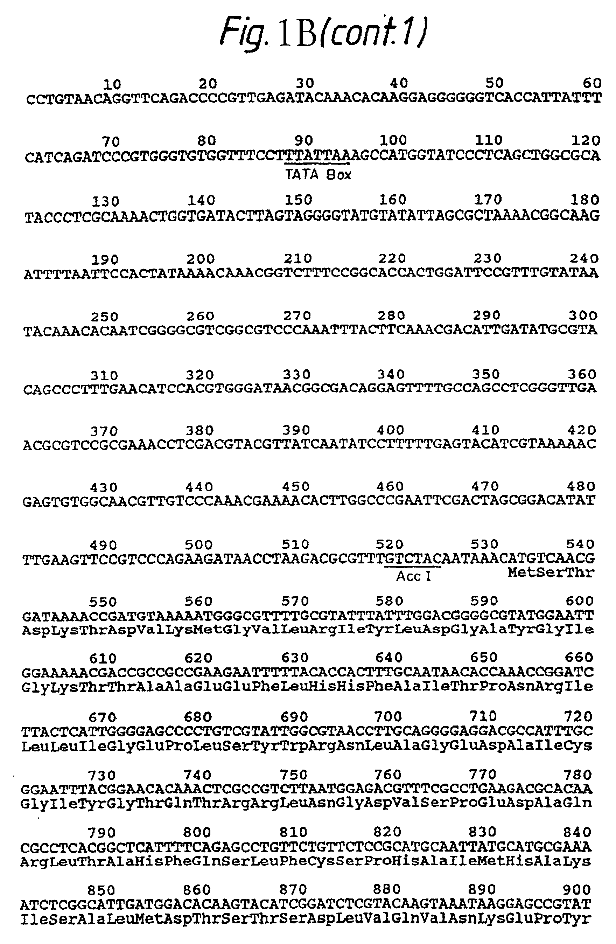

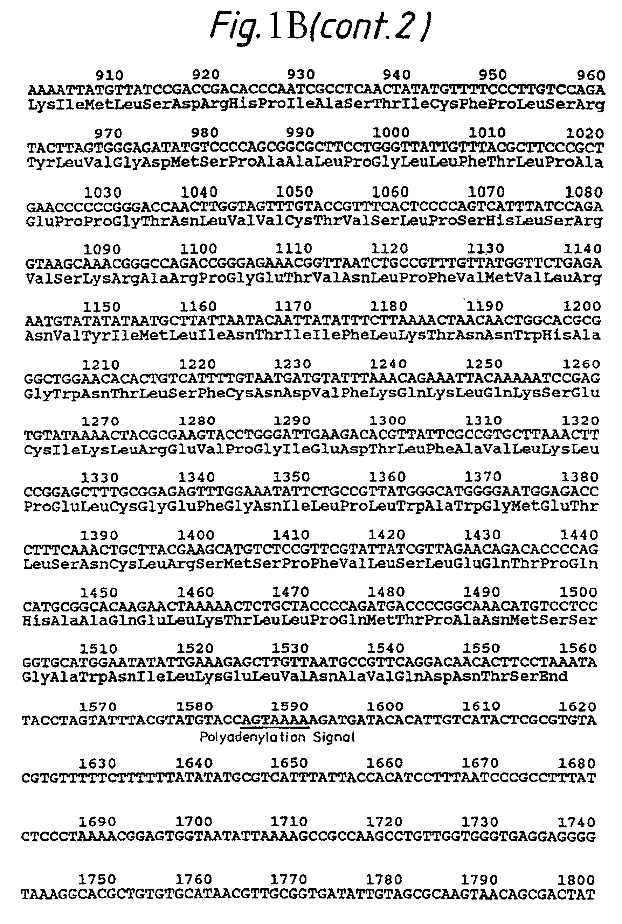

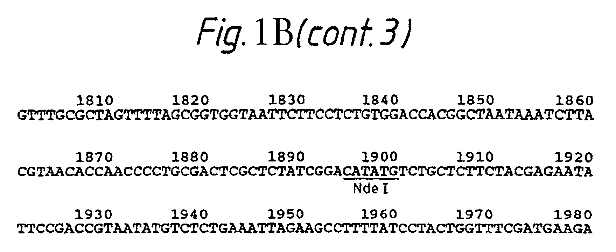

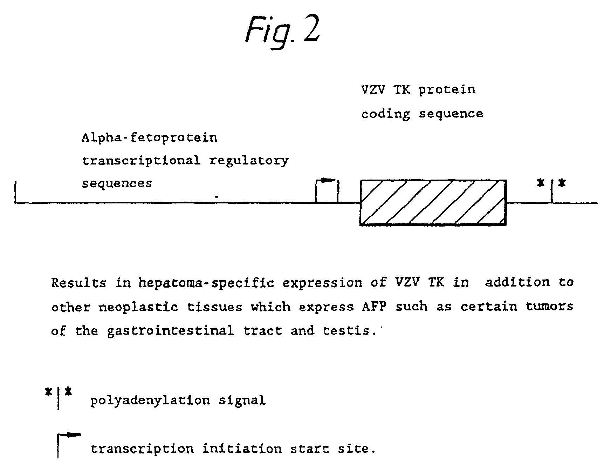

The molecular chimaera of the present invention may be made utilising standard recombinant DNA techniques. Thus the coding sequence and polyadenylation signal of, for example, the VZV thymidine kinase (TK) gene (see Figs. 1A and 1B) is placed in the proper 3' orientation to the essential AFP transcriptional regulatory elements. These molecular chimaeras enable the selective expression of VZV TK in cells which normally express AFP (Figure 2A). Expression of the VZV TK gene in hepatomas, certain tumours of the gastrointestinal tract, nonseminamatous carcinomas of the testis, certain teratocarcinomas and other tumours will enable relatively nontoxic arabinosides and pyrimidines as herein defined to be selectively metabolised to cytotoxic and/or cytostatic metabolites.

Accordingly, in a second aspect, there is provided a method of constructing a molecular chimaera comprising linking a DNA sequence encoding a heterologous enzyme gene, eg. VZV TK to a tissue specific promoter.

In particular the present invention provides a method of constructing a molecular chimaera as herein defined, the method comprising ligating a DNA sequence encoding the coding sequence and polyadenylation signal of the gene for a heterologous enzyme, eg. VZV thymidine kinase to a mammalian tissue specific transcriptional regulatory sequence (eg. promoter sequence and enhancer sequence).

The VZV thymidine kinase coding sequence and 3' polyadenylation signal reside in an approximately 1,381 bp Accl/Nde I restriction endonuclease fragment (see Figure 1).

Preferably it is the 1381 bp Accl/Nde I fragment containing the VZV TK coding sequence and polyadenylation signal that is ligated to the mammalian tissue specific promoter and enhancer sequences, although it will be appreciated that other DNA fragments containing the VZV TK gene could be used. Moreover, the VZV TK polyadenylation signal could be replaced with another suitable polyadenylation signal such as from the SV40 virus or other mammalian genes.

Preferably the promoter and enhancer sequences are selected from the transcriptional regulatory sequences for one of alphafetoprotein (AFP), carcinoembryonic antigen (CEA), or HER2/neu oncogene or other suitable genes.

These molecular chimaeras can be delivered to the target cell by a delivery system. For administration to a patient, it is necessary to provide an efficient *in vivo* delivery system which stably integrates the molecular chimaera into the cells. Known methods utilise techniques of calcium phosphate transfection, electroporation, microinjection, liposomal transfer, ballistic barrage or retroviral infection. For a review of this subject see Biotechnique Vol.6. No.7. 1988.

The technique of retroviral infection of cells to integrate artificial genes employs retroviral shuttle vectors which are known in the art, (see for example Mol. and Cell Biol Aug 86 p2895). Essentially, retorviral shuttle vectors are generated using the DNA form of the retrovirus contained in a plasmid. These plasmids also contain sequences necessary for selection and growth in bacteria. Retroviral shuttle vectors are constructed using standard molecular biology techniques well known in the art. Retroviral shuttle vectors have the parental endogenous retroviral genes (eg. gag, pol and env) removed and the DNA sequence of interest inserted, such as the molecular chimaeras which have been described. They however, contain appropriate retroviral regulatory sequences for viral encapsidation, proviral insertion into the target genome, message splicing, termination and polyadenylation. Retroviral shuttle vectors have been derived from the Moloney murine leukemia virus (Mo-MLV) but it will be appreciated that other retroviruses can be used such as the closely related Moloney murine sarcoma virus. Certain DNA viruses may also prove to be useful as a delivery system. The bovine papilloma virus [BPV] replicates extrachromosomally so that delivery system based on BPV have the advantage that the delivered gene is maintained in a nonintegrated manner.

Thus according to a third aspect of the present invention there is provided a retroviral shuttle vector containing the molecular chimaeras as hereinbefore defined.

The advantages of a retroviral-mediated gene transfer system are the high efficiency of the gene delivery to the targeted tissue, sequence specific integration regarding the viral genome (at the 5' and 3' long terminal repeat (LTR) sequences) and little rearrangements of delivered DNA compared to other DNA delivery systems.

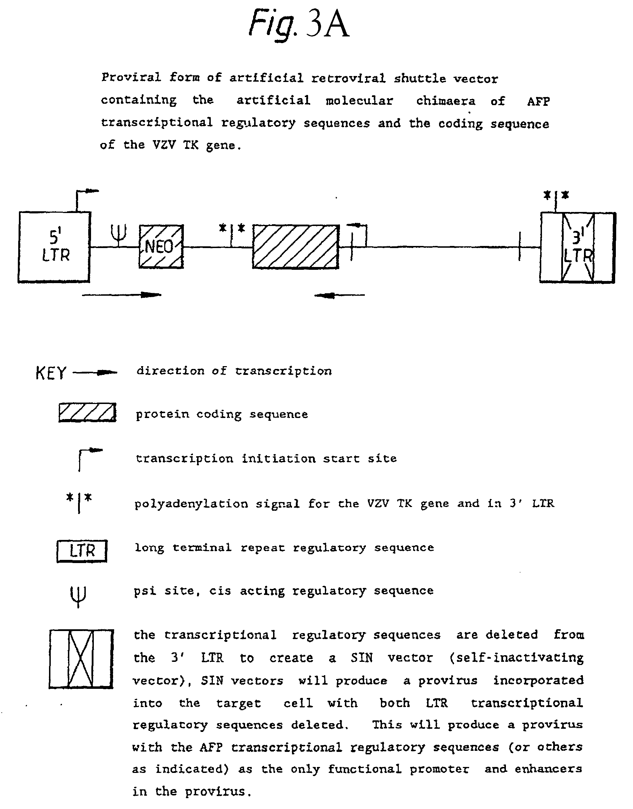

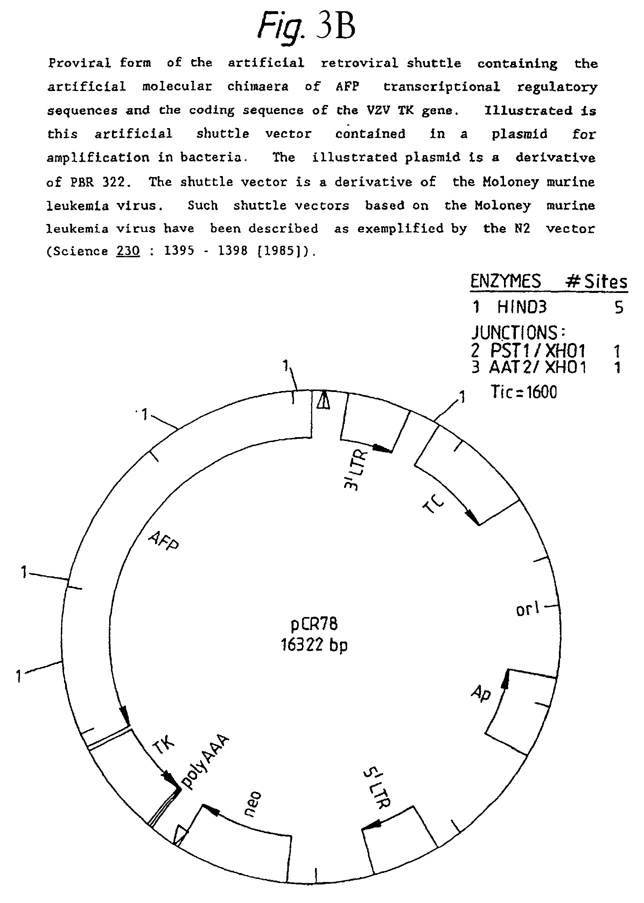

Accordingly in a preferred embodiment of the present invention there is provided a retroviral shuttle vector comprising a DNA sequence comprising a 5' viral LTR sequence, a cis acting psi encapsidation sequence, a molecular chimaera as hereinbefore defined and a 3' viral LTR sequence (figure 3A and figure 3B).

In a preferred embodiment, and to help eliminate non-tissue-specific expression of the molecular chimaera, the molecular chimaera is placed in opposite transcriptional orientation to the 5' retroviral LTR (figure 3A and figure 3B). In addition, a dominant selectable marker gene may also be included which is transcriptionally driven from the 5' LTR sequence. Such a dominant selectable marker gene may be the bacterial neomycin-resistance gene NEO (Aminoglycoside 3' phosphotransferase type II), which confers on eukaroytic cells resistance to the neomycin analogue G418 sulphate (geneticin) (trade mark), (Figure 3A and 3B). The NEO gene aids in the selection of packaging cells which contain these sequences (see below).

The retroviral vector used in the examples is based on the Moloney murine leukemia virus but it will be appreciated that other vectors may be used. Such vectors containing a NEO gene as a selectable marker have been described, for example, the N2 vector (Science 230 : 1395-1398 (1985)).

A theoretical problem associated with retroviral shuttle vectors is the potential of retroviral long terminal repeat (LTR) regulatory sequences transcriptionally activating a cellular oncogene at the site of integration in the host genome. This problem may be diminished by creating SIN vectors (figure 3A). SIN vectors are self-inactivating vectors which contain a deletion comprising the promoter and enhancer regions in the retroviral LTR. The LTR sequences of SIN vectors do not transcriptionally activate 5' or 3' genomic sequences. The transcriptional inactivation of the viral LTR sequences diminishes insertional activation of adjacent target cell DNA sequences and also aids in the selected expression of the delivered molecular chimaera. SIN vectors are created by removal of approximately 299 bp in the 3' viral LTR sequence (Biotechniques 4 504-512 (1986)).

Thus preferably the retroviral shuttle vector of the present invention are SIN vectors.

Since the parental retroviral gag, pol and env genes have been removed from these shuttle vectors, a helper virus system may be utilised to provide the gag, pol and env retroviral gene products trans to package or encapsidate the retroviral vector into an infective virion. This is accomplished by utilising specialised "packaging" cell lines, which are capable of generating infectious, synthetic virus yet are deficient in the ability to produce any detectable wild-type virus. In this way the artificial synthetic virus contains a chimaera of the present invention packaged into synthetic artificial infectious virions free of wild-type helper virus. This is based on the fact that the helper virus that is stably integrated into the packaging cell contains the viral structural genes, but is lacking the psi site, a cis acting regulatory sequence which must be contained in the viral genomic RNA molecule for it to be encapsidated into an infectious viral particle.

Accordingly in a fourth aspect of the present invention there is provided an infective virion comprising a retroviral shuttle vector as hereinbefore described, infective virion comprising a retroviral shuttle vector as hereinbefore described, said vector being encapsidated within viral proteins to create an artificial infective, replication-defective retrovirus.

Preferably the retroviral shuttle vector comprises a shuttle vector comprising a molecular chimaera having the transcriptional regulatory sequence of AFP. In particular, the shuttle vector contains a AFP/VZV TK chimaera.

In addition to removal of the psi site, additional alterations can be made to the helper virus LTR regulatory sequences to ensure that the helper virus is not packaged in virions and is blocked at the level of reverse transcription and viral integration.

Alternatively, helper virus structural genes (i.e gag, pol and env) may be individually and independently transferred into the packaging cell line. Since these viral structural genes are separated within the packaging cell's genome, there is little chance of covert recombinations generating wild-type virus.

In a fifth aspect of the present invention there is provided a method for producing infective virions of the present invention by delivering the artificial retroviral shuttle vector comprising a molecular chimaera of the invention, as hereinbefore described into a packaging cell line.

The packaging cell line may have stably integrated within it a helper virus lacking a psi site and other regulatory sequence as hereinbefore described, or alternatively the packaging cell line may be engineered so as to contain helper virus structural genes within its genome.

The present invention further provides an infective virion as hereinbefore described for use in therapy, particularly for use in the treatment of cancer and more particularly for use in the treatment of HCC, nonseminomatous carcinoma of the testis, certain teratocarcinomas and certain gastrointestinal tumours.

Selective expression of the heterolegous enzyme, in particular thymidine kinase gene is accomplished by utilising tissue-specific, transcriptional regulatory (eg. enhancer and promoter) sequences. Selectivity may be additionally improved by selective infection of liver cells. The retroviral env gene present in the packaging cell line defines the specificity for host infection. The env gene used in constructing the packaging cell line is modified to generate artificial, infective virions that selectively infect hepatocytes. As an example a retroviral env gene introduced into the packaging cell may be modified in such a way that the artificial, infective virion's envelope glycoprotein selectively infect hepatocytes via the specific receptor mediated binding utilised by the hepatitis B virus (HBV).

HBV primarily infects hepatocytes via specific receptor mediated binding. The HBV proteins encoded by the pre-S1 and pre-S2 sequences play a major role in the attachment of HBV to hepatocytes (Hepadna Viruses edited Robinson *et al* 189-203, 205-221, 1987). The env gene of the packaging cell is modified to include the hepatocyte binding site of the large S HBV envelope protein. Such modifications of the env gene introduced into the packaging cell may be performed by standard molecular biology techniques well known in the art and will facilitate viral uptake in the target tissue.

The infective virion according to the invention may be formulated by techniques well known in the art and may be presented as a formulation with a pharmaceutically acceptable carrier therefor. Pharmaceutical acceptable carriers, in this instance, may comprise a liquid medium suitable for use as vehicles to introduce the infective virion into the patient. An example of such a carrier is saline. The infective virion may be a solution or suspension in such a vehicle. Stabilisers and antioxidants and/or other excipients may also be present in such pharmaceutical formulations which may be administered to a mammal by any conventional method eg oral or parenteral routes. In particular, the infective virion may be administered by intra-venous or intra-arterial infusion. In the case of treating HCC intra-hepatic arterial infusion may be advantageous.

Accordingly the invention provides a pharmaceutical formulation comprising an infective virion in admixture with a pharmaceutically acceptable carrier.

Additionally, the present invention provides methods of making pharmaceutical formulations as herein described comprising mixing an artificial infective virion, containing a molecular chimaera, with a pharmaceutically acceptable carrier.

Whilst any suitable compound which can be selectively converted by the enzyme may be utilised, the present invention further provides the use of compounds of formula I or II in the manufacture of a medicament for use in treating cancers capable of expressing VZV thymidine kinase. In particular for use in treating hepatocellular carcinoma (HCC).

6-Substituted purine arabinosides of formula (I) its salts and physiologically functional equivalents thereof as shown hereinbelow: 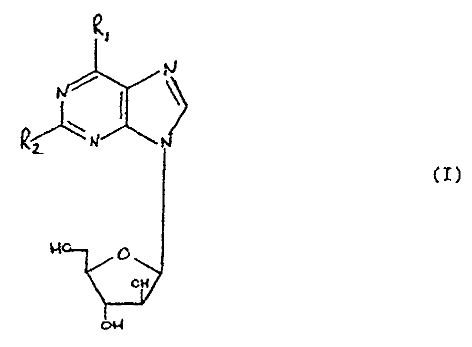 wherein
R₁ is halo, C₁₋₅ alkoxy, halogen-substituted C₁₋₅ alkoxy: an amino group which is mono- or di-substituted by C₁₋₅ alkyl, C₁₋₅ alkyl substituted by one or more fluorine atoms, C₃₋₆ cycloalkyl, or a nitrogen containing heterocycle containing C₄₋₇ carbon atoms and optionally a double bond; and R₂ is hydrogen, halo or amino are known as anti VZV and cytomegalovirus agents and their use and synthesis are described in European patent application published under No.0294114.

Compounds of formula II are as shown hereinbelow 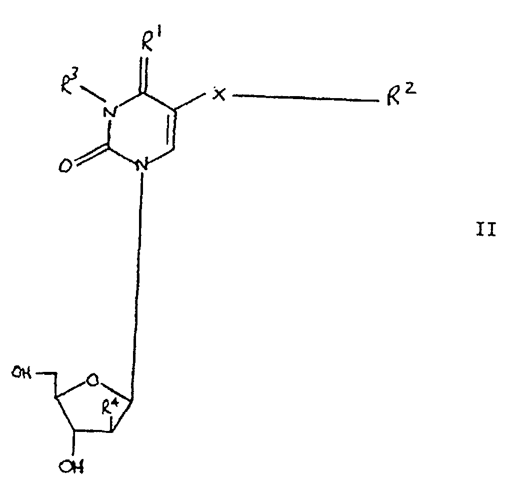 wherein X represents a vinylene or ethynylene group; R¹ represents an oxo or imino group; R² represents a hydrogen atom, C₁₋₂ alkyl, C₃₋₄ branched or cycloalkyl group e.g. isopropyl or cyclopropyl; R³ represents a hydrogen atom or an acyl e.g. C₁₋₄ alkanoyl or benzoyl group optionally substituted for example by one or more halogen, alkyl, hydroxy or alkoxy substituents: and R⁴ represents a hydrogen atom or a hydroxy group; providing that (a) when R², R³ and R⁴ each represents a hydrogen atom, R¹ does not represent an oxo group.

It will be appreciated that when R³ is not an acyl group, the compound of formula (I) or (II) may exist in its tautomeric form. Particularly preferred compounds of formula I and II are 9-β-Arabinofuranosyl-6-methoxy-9H-purine and 1-(β-D-arabinofuranosyl)-5-propynluracil.

These compounds, and methods of their synthesis have been disclosed in European Patent application published under No. 0272065 as having anti VZV activity.

The above-mentioned pyrimidine nucleosides and purine arabinosides also include the pharmaceutically acceptable derivatives of such compounds, ie. any pharmaceutically acceptable salt, ester, or salt of such ester, or any other compound which, upon administration to a human subject, is capable of providing (directly or indirectly) the active metabolite or residue thereof.

Preferably the compound is orally active.

The amounts and precise regime in treating a mammal, will of course be the responsibility of the attendant physician, and will depend on a number of factors including the type and severity of the condition to be treated. However, for HCC, an intrahepatic arterial infusion of the artificial infective virion at a titre of between 2 x 10⁵ and 2 x 10⁷ colony forming units per ml (CFU/ml) infective virions is likely to be suitable for a typical tumour. Total amount of virions infused will be dependent on tumour size and would probably be given in divided doses.

Drug treatment - Subsequent to infection with the infective virion, compounds according to the invention are administered which specifically require VZV TK activity for the critical first phosphorylation step in anabolism to cytotoxic or cytostatic metabolites.

The dose of drug will advantageously be in the range 0.1 to 250 mg per kilogram body weight of recipient per day, preferably 0.1 to 100mg per kilogram bodyweight.

The following examples serve to illustrate the present invention but should not be construed as a limitation thereof:

### Example 1

### Construction of transcriptional regulatory sequence of alphafetoprotein/VZV thymidine kinase

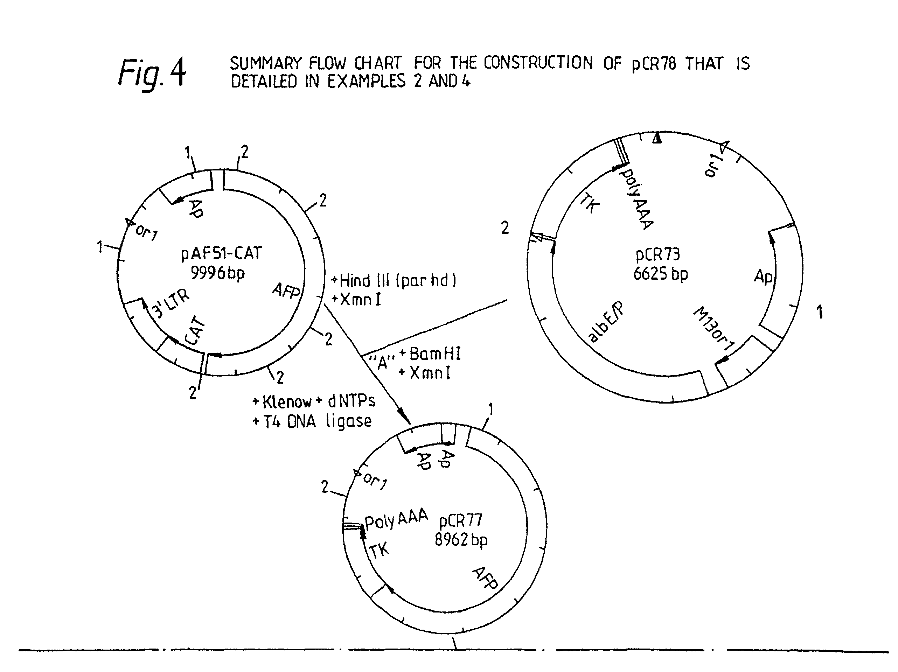

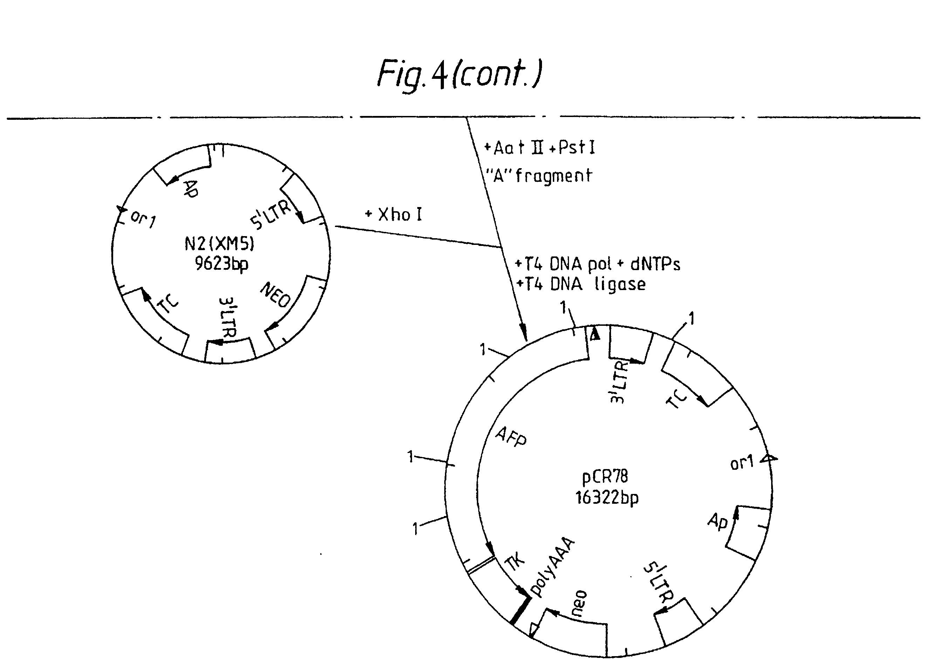

The VZV thymidine kinase coding sequence and polyadenylation site was isolated as an approximately 3,300 bp BamHI-XmnI restriction endonuclease fragment of pCR73 (figure 4). The 5' overhanging end of the BamHI restriction endonuclease site was made blunt by treatment with Klenow and dNTPs. This DNA fragment contains the complete coding sequence and the polyadenylation site of the VZV TK gene but does not contain any enhancer or promoter sequences. An approximately 9996 bp plasmid, pAF5.1-CAT, containing an approximately 5,100 bp of human 5' flanking DNA was obtained from T. Tamaoki, Univ. of Calgary, Canada. A DNA fragment spanning from approximately -5.1 kb to +29 of the human AFP gene was isolated from PAF5.1-CAT by digestion with XmnI and partial digestion with HindIII. This XmnI/HindII fragment was ligated to the BamHI/XmnI VZV TK fragment using T4 DNA ligase to form pCR 77 (figure 4). The AFP E/P VZV TK chimera was purified by electroelution from PCR 77 as an approximately 6699 bp Aat II/PstI restriction endonuclease fragment. This fragment was then treated with T4 DNA polymerase and dNTPS as example 1 to produce a blunt end restriction fragment.

pCR77 was deposited at the ATCC on 18th August 1989, under Accession No. ATCC68079.

### Example 2

### Construction of a retroviral shuttle vector construct containing the molecular chimaera of example 1

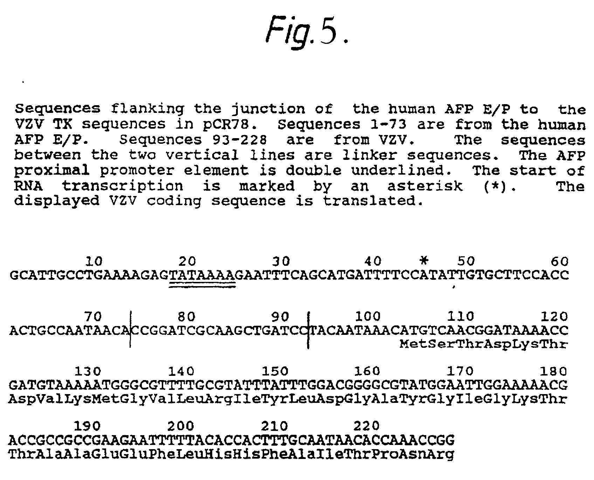

The retroviral shuttle vector pCR78 (figure 4) was constructed by ligating a purified AatII/PstI fragment of pCR77 containing the AFP E/P VZV TK chimaera into a Moloney murine leukemia retroviral vector designated N2(XM5) (Science 230 : 1395-1398, 1985) obtained from S. Karrlson, NIH, Bethesda, MD, USA. N2(XM5) was digested with the restriction endonuclease Xhol and the 5' overhanging ends were made blunt by treatment with both Klenow and dNTPs prior to the ligation to the pCR73 SstI/KpnI fragment using T4 DNA ligase. The retroviral shuttle vector pCR78 containing the AFP E/P VZV TK chimaera has been characterised by restriction endonuclease mapping and DNA sequencing to confirm the primary sequence. The sequence flanking the junction of AFP E/P to the VZV TK sequence is shown in figure 5.

pCR78 was deposited at the ATCC on 18th August 1989 under Accession No. ATCC68080.

### Example 3

### Virus Production

The packaging cell line called PA317 obtained from ATCC, (ATCC CRL 9078), which has been previously described, has three alterations contained within the 5' LTR, psi regulatory sequence and 3' LTR (Mol. and Cell Biol 6 No.8 2895-2902 [1986]). The artificial retroviral constructs described in example 3 are placed into the packaging cell line by electroporation or infection. For electroporation, 20ug of linearized plasmid DNA is electroporated into 2 million PA317 cells in phosphate buffered sucrose using 280 volts, 25 microfarads of capacitance in a total volume of 0.8mls. One can obtain at least 150 G418 resistant colonies / 20ug plasmid DNA/2 million PA317 cells. For infection, 20ug of linearized plasmid DNA is electroporated into 2 million ecotropic packaging cells, such as Psi 2 cells. Two days later, the culture supernatant is used to infect the amphotropic packaging cell line PA317 and 418 resistant colonies isolated. For both electroporation and infection techniques, G418 resistant colonies are single cell cloned by the limiting dilution method and analysed by Southern blots and titered in NIH 3T3 cells (ATCC) to identify the highest producer of full length virus. For PA317 cells containing pCR78, 29 single cell clones were isolated and DNA was obtained from 25 clones. Extensive Southern blot analysis using different restriction endonuclease enzymes and AFP, NEO and VZV TK sequences as radioactive hybridisation probes was performed on these 25 samples. Out of the 25 clones analysed, 5 showed no evidence of truncation and are considered full length. Each packaging cell line containing a full length viral sequence was titered in NIH 3T3 cells which were thymidine kinase minus/minus using standard techniques.

### Example 4

### Infection of a human hepatoma cell line (positive control) with full length infective virions of Example 5 containing AFP/VZV TK with subsequent measurements of VZV Tx activity ara ATP production and drug sensitivity

The replication-defective, full length, artificial retroviruses containing the AFP/VZV TK chimaera were used to infect a human hepatoma cell line called Hep G2 (ATCC KS 8065). Following infection and selection on 1mg geneticin/ml (Trade mark), the cells were assayed for VZV TK activity. In addition, the cells were incubated in the presence of (³H) labeled 9-(B-D-arabinofuranosyl)-6-methoxy-9H purine (designated as ara-M in the following tables and figures) with subsequent measurement of ara ATP. Finally, cells were cultured in the presence of the above mentioned compound and the IC₅₀ - (growth inhibition) was determined. Cells infected with no virus or N2 virus act as control samples for these experiments. N2 virus contain no VZV TK genetic material.

Table 1 demonstrates that the human hepatoma cells infected with pCR78 containing viruses have 52 X more VZV TX activity, respectively, compared to control cells.

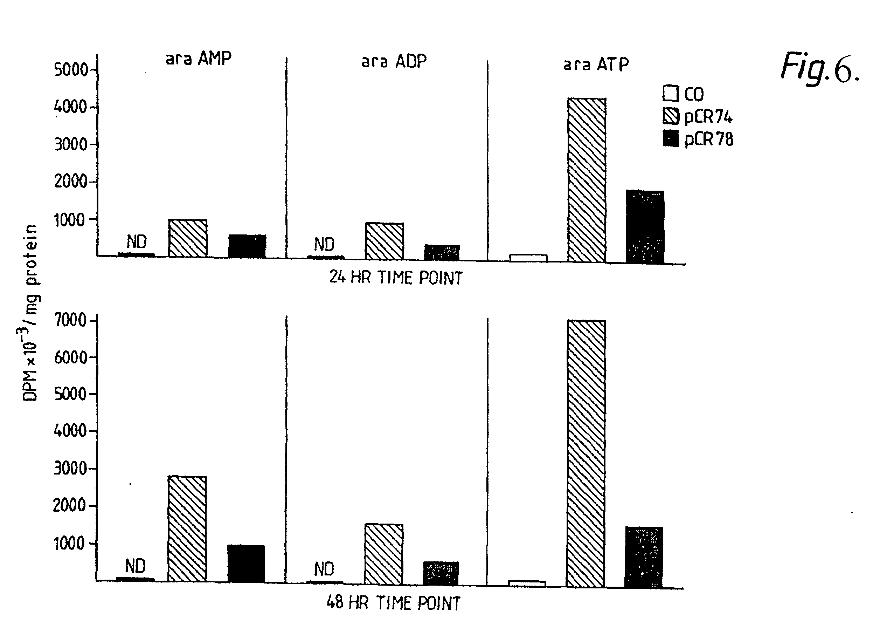

9-(B-D-arabinofuranosyl)-6-methoxy-9H purine (designated as ara-m) can be selectively monophosphorylated by VZV TK with subsequent anabolism to cytotoxic ara ATP. Figure 6 demonstrates that Hep G2 cells which were infected with pCR78 containing viruses and incubated in the presence of (3H) labelled 9-B-D arabinofuranosyl)-6-methoxy-9H purine had significant amounts of cytotoxic (³H) araATP formation.

Hep G2 cells infected with the replication-defective, full length, artificial retroviruses containing the AFP/VZV TK (pCR78) chimaera were incubated in the presence of varying amounts of 9-(B-D-arabinofuranosyl)-6-methoxy-9H purine for 5 days and growth inhibition determined as measured by cell number and DNA content.

Table 2 demonstrates that the IC₅₀ (50% growth inhibition) of 9-(B-D-arabinofuranosyl)-6-methoxy-9H purine is greater than 2000µ M in control and N2 infected Hep G2 cells. Hep G2 cells infected with the replication-defective, full length, artificial retroviruses containing the AFP/VZV TK (pCR78) chimaeras, the IC₅₀ were 175µ M, respectively. Single cell cloning of Hep G2 cells containing the AFP/VZV TK (pCR78) chimaera indicated that the IC₅₀ levels of 9-(B-D-arabinofuranosyl)-6-methoxy-9H purine can be further decreased to approximately 40µ M.

### LEGEND TO FIGURES

Figure 1A: Diagram of Varicella Zoster Thymidine Kinase Gene.
Figure 1B: VZV TK gene - 1° sequence.
Figure 2: Alpha-fetoprotein transcriptional regulatory sequence/VZV TK molecular chimaera.
Figure 3: Proviral form of retrovirus containing alpha-fetoprotein/VZV TK molecular chimaera.
Figure 3B: pCR78.
Figure 4: Flow chart showing the construction of pCR78.
Figure 5: Sequence flanking AFP E/P to VZV TK in pCR78.
Figure 6: Production of ara ATP with cells infected with controls, pCR74, pCR78.

**Table 1**

| VZV TK activity in Hep G2 cells infected with replication-defective, full length, artificial retroviruses containing AFP/VZV TK chimaera (pCR78). VZV Tk activity was quantitated as amount of ara M phosphorylate per mg cellular protein per 30 minutes | | |
|---|---|---|
| Virus | VZV TK Enzymatic Activity nMoles ara MP/µg protein/30 mins | Increase |
| None | 9 | Ox |
| N2 | 4 | Ox |
| pCR78 | 258 | 52x |

**Table 2**

| Growth inhibition in Hep G2 cells infected with replication-defective, full length, artificial retroviruses containing ALB/VZV TK chimaera (pCR74) or AFP/VZV TK chimaera (pCR78). | |
|---|---|
| Virus | IC50 for 9-(B-D-arabinofuranosyl)-6-methoxy-9H-purine |
| None | >2000µM |
| N2 | >2000µM |
| pCR78 | 175µM |

## Claims (Claims for the following Contracting State(s): BE, CH, DE, DK, FR, GB, IT, LU, NL, SE)

1. A molecular chimaera for use in therapy with a prodrug, comprising a cancer specific transcriptional regulatory DNA sequence capable of being selectively activated in a mammalian cancer cell and a DNA sequence operatively linked to the transcriptional regulatory DNA sequence and encoding a heterologous enzyme capable of catalysing the conversion of the prodrug into an agent toxic to the cancer cell.

2. A chimaera according to claim 1 wherein the transcriptional regulatory DNA sequence comprises a promoter.

3. A chimaera according to claim 2, wherein the transcriptional regulatory DNA sequence also comprises an enhancer.

4. A chimaera according to any of the preceding claims, wherein the transcriptional regulatory DNA sequence is derived from the gene for carcinoembryonic antigen, HER2/neu or alphafetoprotein.

5. A chimaera according to any of the preceding claims, additionally comprising a polyadenylation signal downstream of the DNA sequence encoding the heterologous enzyme.

6. A chimaera according to any of the preceding claims, wherein the heterologous enzyme is selected from varicella zoster virus thymidine kinase, carboxypeptidase G2, alkaline phosphatase, penicillin-V amidase and non-mammalian cytosine deaminase.

7. A chimaera according to claim 6, wherein the heterologous enzyme is non-mammalian cytosine deaminase.

8. A chimaera according to any of the preceding claims, wherein the cancer cell is an alphafetoprotein expressing teratocarcinoma or gastrointestinal tumour cell or a testicular non-seminamatous carcinoma cell or hepatoma cell.

9. A viral vector containing a chimaera as claimed in any of the preceding claims.

10. A vector as claimed in claim 9 being a retroviral vector.

11. A vector as claimed in claim 10 being a self inactivating (SIN) vector.

12. A packaging cell line containing a viral vector as claimed in any of claims 9 to 11.

13. An infective virion generated from a packaging cell line as claimed in claim 12.

14. An infective virion as claimed in claim 13, wherein the virion is a retrovirus.

15. An infective virion as claimed in either of claims 13 and 14, wherein the virion is engineered so as to selectively infect a cancer cell.

16. An infective virion as claimed in claim 15, wherein the engineered virion contains a modified envelope glycoprotein.

17. An infective virion as claimed in claim 16, wherein the modified envelope glycoprotein is a modified env gene product containing the hepatocyte binding site of the large S HBV envelope protein.

18. A pharmaceutical formulation comprising an infective virion as claimed in any of claims 13 to 17 and a pharmaceutically acceptable carrier therefor.

19. Use of a prodrug in the manufacture of a medicament for use with a chimaera as claimed in any of claims 1 to 8.

20. Use of a prodrug in the manufacture of a medicament for use with a vector as claimed in any of claims 9 to 11.

21. Use of a prodrug in the manufacture of a medicament for use with an infective virion as claimed in any of claims 13 to 17.

22. Use of a chimaera as claimed in any of claims 1 to 8 in the manufacture of a medicament for use in cancer therapy.

23. Use of a vector as claimed in any of claims 9 to 11 in the manufacture of a medicament for use in cancer therapy.

24. Use of an infective virion as claimed in any of claims 13 to 17 in the manufacture of a medicament for use in cancer therapy.

25. Use according to any of claims 19 to 21, wherein the prodrug is a purine or pyrimidine arabinoside.

26. Use according to claim 25, wherein the prodrug is 9-β-arabinofuranosyl-6-methoxy-9H-purine or 1-(β-D-arabinofuranosyl)-5-propynyluracil or a pharmaceutically acceptable salt, ester or ester salt thereof.

27. Use according to any of claims 19 to 21, wherein the prodrug is para-N-bis(2-chloroethyl)aminobenzoyl glutamic acid, etoposidephosphate, doxorubicin phosphate or mitomycin phosphate, or a phenoxyacetamide derivative of doxorubicin or melphalan.

28. Use according to any of claims 19 to 21, wherein the prodrug is 5-fluorocytosine.

## Claims (Claims for the following Contracting State(s): ES, GR)

1. A process for the production of a molecular chimaera for use in therapy with a prodrug, comprising linking a cancer specific transcriptional regulatory DNA sequence capable of being selectively activated in a mammalian cancer cell and a DNA sequence encoding a heterologous enzyme capable of catalysing the conversion of the prodrug into an agent toxic to the cancer cell.

2. A process according to any of the preceding claims, wherein the transcriptional regulatory DNA sequence comprises a promoter.

3. A process according to claim 2, wherein the transcriptional regulatory DNA sequence also comprises an enhancer.

4. A process according to any of the preceding claims, wherein the transcriptional regulatory DNA sequence is derived from the gene for carcinoembryonic antigen, HER2/neu or alphafetoprotein.

5. A process according to any of the preceding claims, wherein the chimaera additionally comprises a polyadenylation signal downstream of the DNA sequence encoding the heterologous enzyme.

6. A process according to any of the preceding claims, wherein the heterologous enzyme is selected from varicella zoster virus thymidine kinase, carboxypeptidase G2, alkaline phosphatase, penicillin-V amidase and non-mammalian cytosine deaminase.

7. A process according to claim 6, wherein the heterologous enzyme is non-mammalian cytosine deaminase.

8. A process according to any of the preceding claims, wherein the cancer cell is an alphafetoprotein expressing teratocarcinoma or gastrointestinal tumour cell or a testicular non-seminamatous carcinoma cell or hepatoma cell.

9. A process for the production of a retroviral vector containing a molecular chimaera, comprising
ligating DNA sequences containing
a 5' viral LTR sequence,
a cis acting psi encapsidation sequence,
the molecular chimaera produced by the process of any one of claims 1 to 9 and a 3' viral LTR.

10. A process for the production of an infective virion comprising encapsidating a retroviral vector produced by the process of claim 9 into the virion.

11. A process according to claim 10 wherein the infective virion is produced from a packaging cell line.

12. A process for the production of an infective virion produced according to either of claims 9 and 10 and which is capable of selective infection of a target tissue, comprising modifying the env protein of the virion to facilitate cell specific viral uptake.

13. A process for producing a pharmaceutical formulation comprising mixing an infective virion produced according to any of claims 10 to 12 with a pharmaceutically acceptable carrier.

## Patentansprüche (Patentansprüche für folgende(n) Vertragsstaat(en): BE, CH, DE, DK, FR, GB, IT, LU, NL, SE)

1. Molekulare Chimäre zur Verwendung in der Therapie mit einem Prodrug, umfassend eine krebsspezifische Transkriptionsregulations-DNA-Sequenz, die selektiv in einer Säugetier-Krebszelle aktiviert werden kann, und eine DNA-Sequenz, die funktionsfähig mit der Transkriptionsregulations-DNA-Sequenz verbunden ist und ein heterologes Enzym codiert, das die Umwandlung des Prodrugs in ein für die Krebszelle toxisches Mittel katalysieren kann.

2. Chimäre gemäß Anspruch 1, worin die Transkriptionsregulations-DNA-Sequenz einen Promotor umfaßt.

3. Chimäre gemäß Anspruch 2, worin die Transkriptionsregulations-DNA-Sequenz ebenfalls einen Verstärker umfaßt.

4. Chimäre gemäß einem der vorhergehenden Ansprüche, worin die Transkriptionsregulations-DNA-Sequenz aus dem Gen für karzinoembryonisches Antigen, HER2/neu oder alpha-Fetoprotein stammt.

5. Chimäre gemäß einem der vorhergehenden Ansprüche, die zusätzlich ein Polyadenylierungssignal stromabwärts der DNA-Sequenz umfaßt, die das heterologe Enzym codiert.

6. Chimäre gemäß einem der vorhergehenden Ansprüche, worin das heterologe Enzym aus Varicella zoster-Virus-Thymidinkinase, Carboxypeptidase G2, alkalischer Phosphatase, Penicillin-V-Amidase und nicht-Säugetier-Cytosindesaminase ausgewählt ist.

7. Chimäre gemäß Anspruch 6, worin das heterologe Enzym Nicht-Säugetier-Cytosindesaminase ist.

8. Chimäre gemäß einem der vorhergehenden Ansprüche, worin die Krebszelle eine alpha-Fetoprotein-exprimierende Teratokarzinom- oder Magen-Darm-Tumorzelle oder eine testikuläre nicht-seminomatöse Karzinomzelle oder Hepatomzelle ist.

9. Viraler Vektor, der eine Chimäre gemäß einem der vorhergehenden Ansprüche enthält.

10. Vektor gemäß Anspruch 9, der ein retroviraler Vektor ist.

11. Vektor gemäß Anspruch 10, der ein selbstinaktivierender (SIN) Vektor ist.

12. Verpackungszellinie, die einen chiralen Vektor gemäß einem der Ansprüche 9 bis 11 enthält.

13. Infektiöses Virion, das aus einer Verpackungszellinie gemäß Anspruch 12 erzeugt ist.

14. Infektiöses Virion gemäß Anspruch 13, worin das Virion ein Retrovirus ist.

15. Infektiöses Virion gemäß einem der Ansprüche 13 und 14, worin das Virion so konstruiert ist, um selektiv eine Krebszelle zu infizieren.

16. Infektiöses Virion gemäß Anspruch 15, worin das konstruierte Virion ein modifiziertes Hüll-Gylcoprotein enthält.

17. Infektiöses Virion gemäß Anspruch 16, worin das modifizierte Glycoprotein ein modifiziertes env-Genprodukt ist, das die Leberzell-Bindungsstelle des großen S-HBV-Hüllproteins enthält.

18. Pharmazeutische Formulierung, die ein infektiöses Virion gemäß einem der Ansprüche 13 bis 17 und einen pharmazeutisch akzeptablen Träger dafür umfaßt.

19. Verwendung eines Prodrugs in der Herstellung eines Medikaments zur Verwendung mit einer Chimäre gemäß einem der Ansprüche 1 bis 8.

20. Verwendung eines Prodrugs in der Herstellung eines Medikaments zur Verwendung mit einem Vektor gemäß einem der Ansprüche 9 bis 11.

21. Verwendung eines Prodrugs in der Herstellung eines Medikaments zur Verwendung mit einem infektiösen Virion gemäß einem der Ansprüche 13 bis 17.

22. Verwendung einer Chimäre gemäß einem der Ansprüche 1 bis 8 in der Herstellung eines Medikaments zur Verwendung in der Krebstherapie.

23. Verwendung eines Vektors gemäß einem der Ansprüche 9 bis 11 in der Herstellung eines Medikaments zur Verwendung in der Krebstherapie.

24. Verwendung eines infektiösen Virions gemäß einem der Ansprüche 13 bis 17 in der Herstellung eines Medikaments zur Verwendung in der Krebstherapie.

25. Verwendung gemäß einem der Ansprüche 19 bis 21, worin der Prodrug ein Purin- oder Pyrimidinarabinosid ist.

26. Verwendung gemäß Anspruch 25, worin der Prodrug 9-β-Arabinofuranosyl-6-methoxy-9H-purin oder 1-(β-D-Arabinofuranosyl)-5-propinyluracil oder ein pharmazeutisch akzeptables Salz, ein pharmazeutisch akzeptabler Ester oder ein pharmazeutisch akzeptables Estersalz davon ist.

27. Verwendung gemäß einem der Ansprüche 19 bis 21, worin der Prodrug para-N-Bis(2-chlorethyl)aminobenzoylglutaminsäure, Etoposidphosphat, Doxorubicinphosphat oder Mitomycinphosphat oder ein Phenoxyacetamid-Derivat von Doxorubicin oder Melphalan ist.

28. Verwendung gemäß einem der Ansprüche 19 bis 21, worin der Prodrug 5-Fluorocytosin ist.

## Patentansprüche (Patentansprüche für folgende(n) Vertragsstaat(en): ES, GR)

1. Verfahren zur Herstellung einer molekularen Chimäre zur Verwendung in der Therapie mit einem Prodrug, umfassend das Verbinden einer krebsspezifischen Transkriptionsregulations-DNA-Sequenz, die selektiv in einer Säugetier-Krebszelle aktiviert werden kann, und einer DNA-Sequenz, die ein heterologes Enzym codiert, das die Umwandlung des Prodrugs in ein für die Krebszelle toxisches Mittel katalysieren kann.

2. Verfahren gemäß einem der vorhergehenden Ansprüche, worin die Transkriptionsregulations-DNA-Sequenz einen Promotor umfaßt.

3. Verfahren gemäß Anspruch 2, worin die Transkriptionsregulations-DNA-Sequenz ebenfalls einen Verstärker umfaßt.

4. Verfahren gemäß einem der vorhergehenden Ansprüche, worin die Transkriptionsregulations-DNA-Sequenz aus dem Gen für karzinoembryonisches Antigen, HER2/neu oder alpha-Fetoprotein stammt.

5. Verfahren gemäß einem der vorhergehenden Ansprüche, worin die Chimäre zusätzlich ein Polyadenylierungssignal stromabwärts der DNA-Sequenz umfaßt, die das heterologe Enzym codiert.

6. Verfahren gemäß einem der vorhergehenden Ansprüche, worin das heterologe Enzym aus Varizella zoster-Virus-Thymidinkinase, Carboxypeptidase G2, alkalischer Phosphatase, Penicillin-V-Amidase und Nicht-Säugetier-Cytosindesaminase ausgewählt wird.

7. Verfahren gemäß Anspruch 6, worin das heterologe Enzym Nicht-Säugetier-Cytosindesaminase ist.

8. Verfahren gemäß einem der vorhergehenden Ansprüche, worin die Krebszelle eine alpha-Fetoprotein-exprimierende Teratokarzinom- oder Magen-Darm-Tumorzelle oder eine testikuläre nicht-seminomatöse Karzinomzelle der Hepatomzelle ist.

9. Verfahren zur Herstellung eines retroviralen Vektors, der eine molekulare Chimäre enthält, umfassend:
Ligieren von DNA-Sequenzen, die
eine virale 5'-LTR-Sequenz,
eine cis-agierende psi-Enkapsidierungs-Sequenz,
die durch das Verfahren gemäß einem der Ansprüche 1 bis 9 hergestellte molekulare Chimäre und
eine virale 3'-LTR enthalten.

10. Verfahren zur Herstellung eines infektiösen Virions, umfassen das Enkapsidieren eines retroviralen Vektors, der durch das Verfahren des Anspruchs 9 hergestellt wurde, in das Virion.

11. Verfahren gemäß Anspruch 10, worin das infektiöse Virion aus einer Verpackungszellinie erzeugt wird.

12. Verfahren zur Herstellung eines infektiösen Virions, das gemäß einem der Ansprüche 9 und 10 hergestellt wurde und das zur selektiven Infektion eines Zielgewebes fähig ist, umfassend das Modifizieren des env-Proteins des Virions zur Erleichterung der zellspezifischen viralen Aufnahme.

13. Verfahren zur Herstellung einer pharmazeutischen Formulierung, umfassend das Vermischen eines infektiösen Virions, hergestellt gemäß einem der Ansprüche 10 bis 12, mit einem pharmazeutisch akzeptablen Träger.

## Revendications (Revendications pour l'(les) Etat(s) contractant(s) suivant(s): BE, CH, DE, DK, FR, GB, IT, LU, NL, SE)

1. Chimère moléculaire en vue de l'utilisation en thérapie avec une prodrogue, comprenant une séquence d'ADN régulatrice transcriptionnelle spécifique du cancer capable d'être activée de manière sélective dans une cellule cancéreuse mammalienne, et une séquence d'ADN liée de manière opérationnelle à ladite séquence d'ADN régulatrice transcriptionnelle et encodant une enzyme hétérologue capable de catalyser la conversion de la prodrogue en un agent toxique pour la cellule cancéreuse.

2. Chimère selon la revendication 1, dans laquelle la séquence d'ADN régulatrice transcriptionnelle comprend un promoteur.

3. Chimère selon la revendication 2, dans laquelle la séquence d'ADN régulatrice transcriptionnelle comprend aussi un activateur.

4. Chimère selon l'une quelconque des revendications précédentes, dans laquelle la séquence d'ADN régulatrice transcriptionnelle est dérivée du gène pour l'antigène carcino-embryonnaire, le HER2/neu ou l'alphafoetoprotéine.

5. Chimère selon l'une quelconque des revendications précédentes, comprenant en plus, un signal de polyadénylation en aval de la séquence d'ADN encodant l'enzyme hétérologue.

6. Chimère selon l'une quelconque des revendications précédentes, dans laquelle l'enzyme hétérologue est choisie parmi la thymidine-kinase du virus de la varicelle zoster, la carboxypeptidase G2, l'alcaline phosphatase, l'amidase de la pénicilline-V et la cytosine désaminase non-mammalienne.

7. Chimère selon la revendication 6, dans laquelle l'enzyme hétérologue est une cytosine-désaminase non-mammalienne.

8. Chimère selon l'une quelconque des revendications précédentes, dans laquelle la cellule cancéreuse est une cellule tumorale de tératocarcinome ou gastro-intestinale exprimant l'alphafoetoprotéine ou une cellule de carcinome testiculaire non-séminomateux ou une cellule d'hépatome.

9. Vecteur viral contenant une chimère selon l'une quelconque des revendications précédentes.

10. Vecteur selon la revendication 9, qui est vecteur rétroviral.

11. Vecteur selon la revendication 10, qui est un vecteur auto-inactivant (SIN).

12. Lignée cellulaire d'empaquetage contenant un vecteur viral selon l'une quelconque des revendications 9 à 11.

13. Virion infectieux généré à partir d'une lignée cellulaire selon la revendication 12.

14. Virion infectieux selon la revendication 13, lequel virion est un rétrovirus.

15. Virion infectieux selon l'une quelconque des revendications 13 ou 14, lequel virion est construit de manière à infecter sélectivement une cellule cancéreuse.

16. Virion infectieux selon la revendication 15, lequel virion agencé contient une glycoprotéine enveloppe modifiée.

17. Virion infectieux selon la revendication 16, dans lequel la glycoprotéine enveloppe modifiée est un produit env gene contenant le site de liaison d'hépatocyte de la grande protéine enveloppe S HBV.

18. Formulation pharmaceutique comprenant un virion infectieux selon l'une quelconque des revendications 13 à 17, et un véhicule pharmaceutiquement acceptable approprié.

19. Emploi d'une prodrogue dans la fabrication d'un médicament en vue de l'utilisation avec une chimère selon l'une quelconque des revendications 1 à 8.

20. Emploi d'une prodrogue dans la fabrication d'un médicament en vue de l'utilisation avec un vecteur selon l'une quelconque des revendications 9 à 11.

21. Emploi d'une prodrogue dans la fabrication d'un médicament en vue de l'utilisation avec un virion infectieux selon l'une quelconque des revendications 13 à 17.

22. Emploi d'une chimère selon l'une quelconque des revendications 1 à 8, dans la fabrication d'un médicament en vue de l'utilisation dans la thérapie du cancer.

23. Emploi d'un vecteur selon l'une quelconque des revendications 9 à 11, dans la fabrication d'un médicament en vue de l'utilisation dans la thérapie du cancer.

24. Emploi d'une virion infectieux selon l'une quelconque des revendications 13 à 17 dans la fabrication d'un médicament en vue de l'utilisation dans la thérapie du cancer.

25. Utilisation selon l'une quelconque des revendications 19 à 21 dans laquelle la prodrogue est un purine-arabonoside ou un pyrimidine arabinoside.

26. Utilisation selon la revendication 25, dans laquelle la prodrogue est la 9-β-arabinofuranosyl-6-méthoxy-9H-purine ou la 1-(β-D-arabinofuranosyl)-5-propynyluracil ou un de leurs sels, esters, ou sels d'esters pharmaceutiquement acceptable.

27. Utilisation selon l'une quelconque des revendications 19 à 21, dans laquelle la prodrogue est l'acide para-N-bis(2-chloroéthyl)-aminobenzoylglutamique, l'étoposidephosphate, le phosphate de doxorubicine ou le phosphate de mitomycine ou un dérivé phénoxyacétamide de la doxorubicine ou du melphalan.

28. Utilisation selon l'une quelconque des revendications 19 à 21, dans laquelle la prodrogue est la fluorocytosine.

## Revendications (Revendications pour l'(les) Etat(s) contractant(s) suivant(s): ES, GR)

1. Procédé pour la production d'une chimère moléculaire en vue de l'utilisation en thérapie avec une prodrogue, comprenant une séquence d'ADN régulatrice transcriptionnelle spécifique du cancer capable d'être activée de manière sélective dans une cellule cancéreuse mammalienne, et une séquence d'ADN encodant une enzyme hétérologue capable de catalyser la conversion de la prodrogue en un agent toxique pour la cellule cancéreuse.

2. Procédé selon l'une quelconque des revendications précédentes, dans laquelle la séquence d'ADN régulatrice transcriptionnelle comprend un promoteur.

3. Procédé selon la revendication 2, dans lequel la séquence d'ADN régulatrice transcriptionnelle comprend aussi un activateur.

4. Procédé selon l'une quelconque des revendications précédentes, dans lequel la séquence d'ADN régulatrice transcriptionnelle est dérivée du gène pour l'antigène carcino-embryonnaire, le HER2/neu ou l'alphafoetoprotéine.

5. Procédé selon l'une quelconque des revendications précédentes, dans lequel la chimère comprend en plus, un signal de polyadénylation en aval de la séquence d'ADN encodant l'enzyme hétérologue.

6. Procédé selon l'une quelconque des revendications précédentes, dans lequel l'enzyme hétérologue est choisie parmi la thymidine-kinase du virus de la varicelle zoster, la carboxypeptidase G2, l'alcaline phosphatase, la pénicilline-V amidase et la cytosine désaminase non-mammalienne.

7. Procédé selon la revendication 6, dans lequel l'enzyme hétérologue est une cytosine-désaminase non-mammalienne.

8. Procédé selon l'une quelconque des revendications précédentes, dans lequel la cellule cancéreuse est une cellule tumorale de tératocarcinome ou une cellule gastro-intestinale exprimant l'alphafoetoprotéine ou une cellule de carcinome testiculaire non-séminomateux ou une cellule d'hépatome.

9. Procédé pour la production d'un vecteur rétroviral contenant une chimère moléculaire, comprenant
la ligation de séquences d'ADN contenant
une séquence virale LTR 5'
une séquence d'encapsidation psi agissant en cis,
la chimère moléculaire produite par le procédé selon l'une quelconque des revendications 1 à 9 et
une LTR virale 3'.

10. Procédé pour la production d'un virion infectieux comprenant l'encapsidation d'un vecteur rétroviral produit par le procédé selon la revendication 9, à l'intérieur du virion.

11. Procédé selon la revendication 10, dans lequel le virion infectieux est produit à partir d'une lignée cellulaire d'empaquetage.

12. Procédé pour la production d'un virion infectieux produit selon la revendication 9 ou la revendication 10, et qui est capable d'infecter de manière sélective un tissu cible, comprenant la modification de la protéine enveloppe du virion pour faciliter la prise virale spécifique de la cellule.

13. Procédé pour la production d'une formulation pharmaceutique, comprenant le mélange d'un virion infectieux produit selon l'une quelconque des revendications 10 à 12, avec un véhicule pharmaceutiquement acceptable.
